**Europäisches Patentamt**

**European Patent Office**

(19)

**Office européen des brevets**

(11) Veröffentlichungsnummer: **0 170 275**
**B1**

(12) **EUROPÄISCHE PATENTSCHRIFT**

(45) Veröffentlichungstag der Patentschrift:
27.12.89

(21) Anmeldenummer: **85109613.1**

(22) Anmeldetag: **31.07.85**

(51) Int. Cl.⁴: **A 61 K 31/72** //
**A61M1/28**

(54) **Lösungen für die Peritonealdialyse.**

(30) Priorität: 31.07.84 DE 3428201

(43) Veröffentlichungstag der Anmeldung:
05.02.86 Patentblatt 86/6

(45) Bekanntmachung des Hinweises auf die Patenterteilung:
27.12.89 Patentblatt 89/52

(84) Benannte Vertragsstaaten:
AT BE CH DE FR GB IT LI LU NL SE

(56) Entgegenhaltungen:
EP-A-0 076 355
WO-A-83/00087
DD-A-135 352
DE-A-2 837 067
DE-A-3 030 863
DE-B-2 201 669
GB-A-1 583 006
GB-A-2 132 914
US-A-3 523 938

(73) Patentinhaber: **Laevosan- Gesellschaft m.b.H.,**
**Estermannstrasse 17, A-4020 Linz (AT)**

(72) Erfinder: **Kramar, Reinhard, Dr. med.,**
**Wüstenrotstrasse 5, D-4020 Linz (DE)**
Erfinder: **Ferber, Hubert, Dr. med., Ahornweg 24,**
**D-4052 Ansfelden (DE)**

(74) Vertreter: **Huber, Bernhard, Dipl.- Chem.,**
**Patentanwälte H. Weickmann, Dr. K. Fincke**
**F.A. Weickmann, B. Huber Dr. H. Liska, Dr. J.**
**Prechtel Möhlstrasse 22 Postfach 860 820, D-8000**
**München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

2

**Beschreibung**

Die Erfindung betrifft ein Verfahren zur Herstellung eines Peritonealdialysemittels, das in einem an sich bekannten, für die Peritonealdialyse geeigneten Medium eine osmotische Trägersubstanz enthält.

Für die Peritonealdialyse, z. B. die continuierliche ambulante Peritonealdialyse (CAPD) oder die continuierliche Cycling-Peritonealdialyse (CCPD) (vgl. "Praxis der CAPD", herausgegeben von F. Scheler und C. Fuchs, Bibliomed, Medizinische Verlagsgesellschaft mbH Melsungen, 1981, S. 1 - 139) sind derzeit Dialyselösungen auf der Basis von Glucose als osmotischer Trägersubstanz gebräuchlich. Solche Lösungen besitzen jedoch den Nachteil, daß sie resorbiert werden können; dadurch kann der Glucosespiegel erhöht werden, was insbesondere dann sehr nachteilig ist, wenn Störungen des Zukkerstoffwechsels vorliegen.

Es bestanden deshalb schon seit längerer Zeit Bestrebungen, in den Dialyselösungen die Glucose durch andere Stoffe zu ersetzen, bei denen keine unerwünschte Resorption auftritt, und mit denen auch eine längere Verweilzeit und damit ein Wechsel in größeren Zeitabschnitten möglich ist. Als Ersatzstoffe für die Glucose wurden Polyanionen und Glycerin in die engere Wahl gezogen; Polyanionen besitzen jedoch den Nachteil, daß durch sie Veränderungen in der Membranporengröße auftreten können, die sich auf die Dialyse negativ auswirken; die Arbeiten mit Polyanionen sind deshalb bisher nicht über das Tierversuchsstadium hinaus fortgeschritten. Auch gegen den Einsatz von Glycerin bestehen erhebliche Bedenken: Glycerin besitzt eine nephrotoxische Wirkung und kann, weil es aufgrund des niedrigen Molekulargewichts resorbierbar ist, die Restnierenfunktion stören.

Aufgabe der vorliegenden Erfindung ist deshalb die Bereitstellung einer Dialyselösung für die Peritonealdialyse, die die bekannten Nachteile solcher Lösungen nicht aufweist. Diese Aufgabe wird mit den erfindungsgemäßen Dialyselösungen gelöst.

Gegenstand der vorliegenden Erfindung ist daher ein Verfahren zur Herstellung eines Peritonealdialysemittels, das in einem an sich bekannten, für die Peritonealdialyse geeigneten Medium eine osmotische Trägersubstanz enthält, das dadurch gekennzeichnet ist, daß als osmotische Trägersubstanz Hydroxyäthylstärke mit einem Molekulargewicht $\geq 3 \times 10^4$ Dalton und einem Substitutionsgrad von 0,25 bis 0,70 verwendet wird.

Als Medium kann z. B. jedes für die Peritonealdialyse, insbesondere für die Peritonealdialyse mit Glucose als osmotischer Trägersubstanz übliches Medium verwendet werden, z. B. eine Ringer-Lösung.

Durch die Verwendung einer Hydroxyäthylstärke mit einem Molekulargewicht von $\geq 3 \times 10^4$ Dalton wird sichergestellt, daß keine Resorption mehr stattfindet. Der obere Grenzwert des Molekulargewichts ist nicht kritisch; er ergibt sich aus der Art und Herstellung der Hydroxyethylstärke.

Mit der erfindungsgemäß hergestellten Lösung für die Peritonealdialyse ist es möglich, die Nachteile (insbesondere Resorption) der bekannten Dialyselösungen zu vermeiden; der Übertritt von Lösung durch die Membran läßt sich unter den Bedingungen einer Verminderung des colloidosmotischen Druckes länger aufrechterhalten, wodurch die Verweilzeit (Anwendungsdauer) der Dialyselösung erhöht wird.

Der Substitutionsgrad (Mol Hydroxyäthyläthergruppen/Mol Anhydroglucose) liegt vorzugsweise zwischen 0,4 und 0,6 und insbesondere bei ca. 0,5.

Als Hydroxyäthylstärke kann jede Hydroxyäthylstärke mit dem geeigneten Molekulargewicht und Substitutionsgrad eingesetzt werden. Die Herstellung einer solchen Hydroxyäthylstärke kann z. B. auf an sich bekannte Weise durch hydrolytischen und/oder enzymatischen Abbau einer Stärke, insbesondere einer an Amylopektin reichen Stärke, auf ein bestimmtes Molekulargewicht und partielle Verätherung bis zum gewünschten Substitutionsgrad erfolgen (vgl. z. B. US-PS-3 523 938, DE-OS-2 837 067). Ein enzymatischer Abbau erfolgt vorzugsweise mit α oder ß-Amylase oder mittels Pullulanase (vgl. deutsche Patentanmeldung P-3 313 600.9). Zur Herstellung der Dialyselösung kann eine feste, z. B. durch Sprühtrocknung oder Vakuumtrocknung gewonnene Hydroxyäthylstärke eingesetzt werden, oder aber auch die nach der Reinigung, insbesondere Diafiltration erhaltenen Hydrolyselösungen.

Die erfindungsgemäß hergestellte Lösung zur Peritonealdialyse enthält die Hydroxyäthylstärke vorzugsweise in einer Menge von 3 bis 10 Gew.-%, bezogen auf die fertige Lösung.

Neben der Hydroxyäthylstärke als osmotische Trägersubstanz können die erfindungsgemäß hergestellten Lösungen zur Peritonealdialyse noch weitere Substanzen enthalten, insbesondere die neben der osmotischen Trägersubstanz üblichen Bestandteile von Peritonealdialyse-Lösungen, wie z. B. die Bestandteile der Ringer-Lösung. Eine mit dem erfindungsgemäßen Verfahren bevorzugt hergestellte Dialyselösung enthält z. B. 3 bis 10 Gew.-% Hydroxyäthylstärke, 125 bis 150 mMol/l Natriumionen, 90 bis 110 mMol/l Chloridionen, 0,3 bis 1,5 mMol/l Magnesiumionen, 1,0 bis 2,5 mMol/l Calciumionen und 35 bis 45 mMol/l Lactat. Das Lactat kann auch ganz oder teilweise durch Acetat ersetzt sein. Die genannten Kationen und Anionen liegen dabei vorzugsweise als Salz der ebenfalls als Bestandteile genannten Gegenionen vor; es kommen aber, insbesondere bei einer mangelnden Menge an Gegenionen, auch andere physiologisch verträgliche Kationen bzw. Anionen in Frage.

Als weitere Bestandteile können nach dem erfindungsgemäßen Verfahren hergestellte Dialyselösungen eine oder mehrere pharmakologisch aktive Substanzen enthalten, wie sie z. B. auch in bisher üblichen Dialyselösungen verwendet werden. Solche Wirkstoffe sind insbesondere Vasodilatatoren, wie z. B. Natriumnitroprussid, Diuretika, Hormone, wie z. B. Insulin und/oder Vitamine, wie z. B. insbesondere Vitamin E. Die Art und Menge dieser Wirkstoffe richtet sich dabei insbesondere nach dem jeweiligen Einzelfall.

Als weitere Bestandteile können den Dialyselösungen zugesetzt werden:

Glucose, vorzugsweise in einer Menge von 0,1 bis 4 Gew.-%, bezogen auf die fertige Lösung;

physiologisch verträgliche Aminosäuren und-/oder deren korrespondierende Ketosäuren, insbesondere die für den Eiweißstoffwechsel wichtigen und körpereigenen Aminosäuren bzw. deren korrespondierende Ketosäuren.

Ein Zusatz an Glucose und/oder Aminosäuren bzw. deren korrespondierende Ketosäuren ist insbesondere dann zweckmäßig, wenn mit der Dialyselösung auch eine gewisse Alimentierung (Nahrungszufuhr) beabsichtigt wird.

Die Menge der neben der Hydroxyäthylstärke vorhandenen Bestandteile ist insbesondere vom Gehalt an Hydroxyäthylstärke abhängig, die Art und Menge der pharmakologisch wirksamen Substanzen richtet sich insbesondere nach dem jeweiligen Einzelfall.

Die nach dem erfindungsgemäßen Verfahren hergestellten Dialyselösungen können durch Auflösen der Bestandteile in destilliertem Wasser und anschließende Sterilisation hergestellt werden. Die Bestandteile können dabei gleichzeitig gelöst werden; es kann aber auch stufenweise vorgegangen werden, indem man einen oder mehrere Bestandteile nacheinander auflöst; z. B. kann die Hydroxyäthylstärke und gegebenenfalls weitere Bestandteile in einer fertigen Ringer-Lösung oder einem anderen für derartige Dialyselösungen geeigneten Medium aufgelöst werden.

Die Dialyselösung wird in für die Peritonealdialyse üblichen und bekannten Behältern aufbewahrt und verwendet, die sowohl aus starrem (z. B. Glas) oder aber auch aus biegsamem Material (Kunststoffe), und insbesondere aus dünnen zusammenfaltbaren Kunststoffolien bestehen können.

Vorteilhafterweise wird die Herstellung und Sterilisation schon in den zur Aufbewahrung und Verwendung geeigneten Behältern durchgeführt. In verschiedener Hinsicht (Lagerung, Transport, Stabilität der Lösungen, Wahl der Dialyselösung je nach Zweckbestimmung) kann es aber auch zweckmäßig sein, einige oder alle Bestandteile der Dialyselösung in fester Form, z. B. in lyophilisierter Form, in die Dialysebehälter abzufüllen und erst kurz vor ihrer Verwendung in dem geeigneten Medium, z. B. in destilliertem Wasser oder Ringer-Lösung, aufzulösen.

Das nachfolgende Beispiel zeigt eine typische Zusammensetzung einer erfindungsgemäßen Lösung zur Peritonealdialyse:

**Beispiel**

Hydroxyäthylstärke 200/0,5 (Gewichtsmittel des Molekulargewichts ca. 200 000 Dalton, molare Substitution 0,5 Mol Hydroxyäthyl-äthergruppen/Mol Anhydroglucose     70 g

| | |
|---|---|
| Na$^+$ (als Chlorid und Lactat) | 132 mMol |
| Cl$^-$ (als Natriumsalz) | 102 mMol |
| Mg$^{++}$ (als Lactat) | 0,5 mMol |
| Ca$^{++}$ (als Lactat) | 1,75 mMol |
| Lactat | 40 mMol |
| destilliertes Wasser | ad 1000 ml. |

**Patentansprüche**

1. Verfahren zur Herstellung eines Peritonealdialysemittels, das in einem an sich bekannten, für die Peritonealdialyse geeigneten Medium eine osmotische Trägersubstanz enthält, *dadurch gekennzeichnet*, daß als osmotische Trägersubstanz Hydroxyäthylstärke mit einem Molekulargewicht $\geq 3 \times 10^4$ Dalton und einem Substitutionsgrad von 0,25 bis 0,70 verwendet wird.

2. Verfahren nach Anspruch 1, *dadurch gekennzeichnet*, daß man eine Hydroxyäthylstärke mit einem Substitutionsgrad von 0,4 bis 0,6 verwendet.

3. Verfahren nach Anspruch 1 oder 2, *dadurch gekennzeichnet*, daß die Hydroxyäthylstärke in einer Menge von 3 bis 10 Gew.-%, bezogen auf das fertige Mittel, verwendet wird.

4. Verfahren nach einem der Ansprüche 1 bis 3, *dadurch gekennzeichnet*, daß ein Peritonealdialysemittel mit 3 bis 10 Gew.-% Hydroxyäthylstärke, 125 bis 150 mMol/l Natriumionen, 90 bis 110 mMol/l Chloridionen, 0,3 bis 1,5 mMol/l Magnesiumionen, 1,0 bis 2,5 mMol/l Calciumionen und 35 bis 45 mMol/l Lactat, wobei das Lactat ganz oder teilweise durch Acetat ersetzt sein kann, hergestellt wird.

5. Verfahren nach einem der Ansprüche 1 bis 4, *dadurch gekennzeichnet*, daß dem Peritonealdialysemittel als weitere Bestandteile eine oder mehrere pharmakologisch wirksame Substanzen zugesetzt werden.

6. Verfahren nach Anspruch 5, *dadurch gekennzeichnet*, daß als pharmakologisch aktive Substanzen Vasodilatatoren, Diuretika, gerinnungshemmende Substanzen, Antibiotika, Chemotherapeutika, Hormone und/oder Vitamine verwendet werden.

7. Verfahren nach einem der Ansprüche 1 bis 6, *dadurch gekennzeichnet*, daß als weiterer Bestandteil 0,1 bis 4 Gew.-%, bezogen auf das fertige Peritonealdialysemittel, Glucose zugesetzt wird.

8. Verfahren nach einem der Ansprüche 1 bis 7, *dadurch gekennzeichnet*, daß als weitere Bestandteile eine oder mehrere Aminosäuren und/oder deren korrespondierende Ketosäuren zugesetzt werden.

9. Verfahren nach einem der vorhergehenden Ansprüche, *dadurch gekennzeichnet*, daß man die Bestandteile in destilliertem Wasser auflöst und sterilisiert.

10. Verfahren nach Anspruch 9, *dadurch gekennzeichnet*, daß man die Peritonealdialyselösung stufenweise durch aufeinanderfolgendes Auflösen von einem oder mehreren Bestandteilen herstellt.

## Claims

1. Process for the preparation of a peritoneal dialysis agent which contains an osmotic carrier substance in a per se known medium suitable for peritoneal dialysis, characterised in that, as osmotic carrier substance, there is used hydroxyethylstarch with a molecular weight of $\geq 3 \times 10^4$ Dalton and a degree of substitution of 0.25 to 0.70.

2. Process according to claim 1, characterised in that one uses a hydroxyethylstarch with a degree of substitution of 0.4 to 0.6.

3. Process according to claim 1 or 2, characterised in that the hydroxyethylstarch is used in an amount of 3 to 10 wt.-%, referred to the final agent.

4. Process according to one of claims 1 to 3, characterised in that a peritoneal dialysis agent is prepared with 3 to 10 wt.% hydroxyethylstarch, 125 to 150 mMol/l. sodium ions, 90 to 110 mMol/l. chloride ions, 0.3 to 1.5 mMol/l. magnesium ions, 1.0 to 2.5 mMol/l. calcium ions and 35 to 45 mMol/l. lactate, whereby the lactate can be replaced wholly or partly by acetate.

5. Process according to one of claims 1 to 4, characterised in that to the peritoneal dialysis agent is added, as further components, one or more pharmacologically-active substances.

6. Process according to claim 5, characterised in that, as pharmacologically-active substances, there are used vasodilators, diuretics, coagulation-inhibiting substances, antibiotics, chemotherapeutics, hormones and/or vitamins.

7. Process according to one of claims 1 to 6, characterised in that, as further component, there is added 0.1 to 4 wt.-%, referred to the final peritoneal dialysis agent, of glucose.

8. Process according to one of claims 1 to 7, characterised in that, as further components, there are added one or more amino acids and/or their corresponding keto acids.

9. Process according to one of the preceding claims, characterised in that one dissolves the components in distilled water and sterilises.

10. Process according to claim 9, characterised in that one prepares the peritoneal dialysis solution stepwise by successive dissolving of one or more components.

## Revendications

1. Procédé de préparation d'un moyen pour dialyse péritonéale qui contient une substance véhicule osmotique dans un milieu connu en soi, approprié pour la dialyse péritonéale, caractérisé en ce que l'on utilise en tant que substance véhicule osmotique de l'hydroxyéthylamidon ayant un poids moléculaire $\geq 3 \times 10^4$ Daltons et un degré de substitution de 0,25 à 0,70.

2. Procédé selon la revendication 1, caractérisé en ce que l'on utilise un hydroxyéthylamidon ayant un degré de substitution de 0,4 à 0,6.

3. Procédé selon la revendication 1 ou 2, caractérisé en ce que l'hydroxyéthylamidon est utilisé en une quantité de 3 à 10 % en poids par rapport au moyen terminé.

4. Procédé selon l'une des revendications 1 à 3, caractérisé en ce que l'on prépare un moyen pour dialyse péritonéale avec 3 à 10 % en poids d'hydroxyéthylamidon, 125 à 150 mmol/l d'ions sodium, 90 à 110 mmol/l d'ions chlorure, 0,3 à 1,5 mmol/l d'ions magnésium, 1,0 à 2,5 mmol/l d'ions calcium et 35 à 45 mmol/l de lactate, le lactate pouvant être totalement ou partiellement remplacé par l'acétate.

5. Procédé selon l'une des revendications 1 à 4, caractérisé en ce qu'une ou plusieurs substances actives du point pharmacologique sont ajoutées au moyen pour dialyse péritonéale en tant qu'autres constituants.

6. Procédé selon la revendication 5, caractérisé en ce que, comme substances actives du point de vue pharmacologique, sont utilisés des vasodilatateurs, des diurétiques, des substances anticoagulantes, des antibiotiques, des substances chimiothérapeutiques, des hormones et/ou des vitamines.

7. Procédé selon l'une des revendications 1 à 6, caractérisé en ce que 0,1 à 4 % en poids de glucose, par rapport au moyen pour dialyse péritonéale terminé, sont ajoutés en tant qu'autre constituant.

8. Procédé selon l'une des revendications 1 à 7, caractérisé en ce qu'un ou plusieurs acides aminés et/ou acides cétoniques correspondants sont ajoutés en tant qu'autres constituants.

9. Procédé selon l'une des revendications précédentes, caractérisé en ce que l'on dissout et stérilise les constituants dans l'eau distillée.

10. Procédé selon la revendication 9, caractérisé en ce que l'on prépare la solution pour dialyse péritonéale par étapes par dissolution consécutive d'un ou plusieurs constituants.